# EUROPEAN PATENT APPLICATION

(11) **EP 3 135 241 A1**
(43) Date of publication of application: **01.03.2017**
(21) Application number: 15182446.3
(22) Date of filing: 26.08.2015
(51) Int. Cl.: A61C 9/00, A61K 6/10, A61C 19/06, A61F 5/56

(54) **DENTAL TRAY MOLDING KITS AND METHODS**

(71) Applicant: Ackel, Gilbert, Cote-St-Luc, Quebec H4V 2G8 (CA)
(72) Inventor: Ackel, Gilbert, Cote-St-Luc, Quebec H4V 2G8 (CA)
(74) Representative: Delorme, Nicolas

(57) **Abstract**

A dental tray molding kit is provided. The kit includes a moldable generic tray and a thermoformable material removably insertable in the moldable tray and biteable by a user upon placement of the thermoformable material inserted into the moldable tray in a mouth of the user. Another dental tray molding kit is also provided. The kit includes a rigid generic tray; an impression material removably insertable in the rigid tray and biteable by a user upon placement of the impression material inserted into the rigid tray in a mouth of the user to create an impression tray; and a thermoformable material removably insertable in the impression tray and biteable by the user upon placement of the thermoformable material inserted into the impression tray in the mouth. Methods for using the kits are also provided. The kits can also be configured to whiten teeth of the user as a cosmetic procedure.

## Description

### FIELD OF THE INVENTION

The present invention relates to dental tray molding kits and methods for cosmetic teeth whitening.

### BACKGROUND OF THE INVENTION

Many dental health care professionals, such as dentists, use dental tray molding techniques for various dental procedures.

In certain cases, molding a dental tray can be indispensable. However, techniques known in the art fail to provide a thin, durable, moldable tray that is as close as possible to a dental laboratory fabricated tray. Known techniques are generally time consuming to produce as they require sending dental impressions to a dental laboratory.

Some trays are also too bulky and are poorly fitted to a patient's teeth. These bulky trays are also easily noticed which can make certain patients uncomfortable. Additionally, bulky trays can be quite problematic in procedures requiring the use of gels, as bulky trays are often also poorly fitted. In these events, the poorly fitted trays allow the gels to leak out which can render the procedure less efficient. Poorly fitted trays are also poorly adapted for holding onto a patient's teeth and can be quite uncomfortable and inconvenient for the patient.

Also some trays are made of materials that are not properly suited for some dental procedures. For instance, some trays are ill-fitted or poorly adapted to be used as bruxism devices.

Thus, known dental tray molding methods and kits lack the full set of attributes needed for easily fabricating a custom fitted dental tray quickly, accurately and at relatively low-cost for various dental procedures. There therefore exists a need in the art for dental tray molding methods and kits which alleviates at least some of the drawbacks of the prior art.

### SUMMARY OF THE INVENTION

According to an aspect of the invention, there is provided a dental tray molding kit. The kit includes a generic tray and a thermoformable material removably insertable in the generic tray and biteable by a user upon placement of the thermoformable material inserted into the generic tray in a mouth of the user.

In some embodiments, the generic tray is a moldable generic tray.

Preferably, the moldable generic tray is made of an ethylene vinyl-acetate material. Also preferably, the generic tray is also typically sufficiently pliable for receiving a dental impression.

Preferably, the thermoformable material is a thermoplastic material. Also preferably, the thermoplastic material is a polyester material. In some embodiments, the thermoformable material can be of a cylindrical shape.

In some embodiments, the kit can further include instructions. The instructions can be a dental tray molding method that includes the steps of:
- providing a recipient of hot water sized to receive a moldable generic tray;
- submerging the moldable generic tray to the hot water until sufficiently pliable;
- inserting the moldable generic tray into a mouth;
- biting the moldable generic tray with suction to mold a first custom impression tray;
- removing the first custom impression tray from the mouth to cool;
- submerging a thermoformable material to the hot water until sufficiently pliable;
- placing the thermoformable material into the first custom impression tray;
- inserting the first custom impression tray with the thermoformable material in the mouth;
- biting the thermoformable material within the first custom impression tray using pumping action to create a second custom impression tray;
- removing the first and the second custom impression trays from the mouth to cool; and
- separating the second custom impression dental tray from the first custom impression tray.

Preferably, the kit is configurable to whiten teeth of the user as a cosmetic procedure.

In other embodiments of the kit, the generic tray is a rigid generic tray and an impression material is removably insertable in the rigid generic tray and biteable by a user upon placement of the impression material inserted into the rigid generic tray in a mouth of the user to create a custom impression tray. A thermoformable material is removably insertable in the custom impression tray and biteable by the user upon placement of the thermoformable material inserted into the custom impression tray in the mouth.

In some embodiments, the kit can further include instructions. The instructions can be a dental tray molding method that includes the steps of:
- inserting impression material into a rigid generic tray;
- inserting the rigid generic tray with the impression material into a mouth;
- biting the impression material within the rigid generic tray with suction to create a first custom impression tray;
- submerging a thermoformable material to the hot water until sufficiently pliable;
- placing the thermoformable material into the first custom impression tray;
- inserting the first custom impression tray with the thermoformable material in the mouth;
- biting the thermoformable material within the first custom impression tray using pumping action to create a second custom impression tray;
- removing the first and the second custom impression trays from the mouth to cool; and
- separating the second custom impression tray from the first custom impression tray.

Preferably, the kit is configurable to whiten teeth of the user as a cosmetic procedure.

Advantageously, the dental tray molding method according to embodiments of the invention allows for a well-adapted custom mouth tray that holds the thermoformable material in place while creating the second custom dental tray and allows for easy manipulation of the thermoformable material for creating a thin, durable second custom dental tray having a precise dental fit.

Also advantageously, the dental tray molding method according to embodiments of the invention can be easily fabricated in a few minutes without having to send a dental impression to a laboratory. The dental tray created is also generally thin and aesthetic and is not as noticeable as bulky dental trays. It is also well adapted to hold onto a user's teeth without requiring support or additional handling.

Other features and advantages of the present invention will be better understood upon a reading of embodiments thereof with reference to the appended drawings.

### BRIEF DESCRIPTION OF THE DRAWINGS

Figure 1 schematically illustrates a dental tray molding kit using, in accordance with an embodiment of the invention.
Figure 2 is a flow chart of a dental tray molding method, in accordance with an embodiment of the invention.
Figure 3 schematically illustrates another dental tray molding kit using, in accordance with an embodiment of the invention.
Figure 4 is a flow chart of another dental tray molding method, in accordance with an embodiment of the invention.

### DESCRIPTION OF PREFERRED EMBODIMENTS OF THE INVENTION

In the following description, similar features in the drawings have been given similar reference numerals and in order to weigh down the figures, some elements cannot be referred to on some figures if they were already identified in preceding figures. It should also be understood herein that the elements of the drawings are not necessarily drawn to scale and that the emphasis is instead being placed upon clearly illustrating the elements and structures of the present embodiments.

It is also to be understood that certain descriptions of the present invention have been simplified to illustrate only those elements and limitations that are relevant to a clear understanding of the present invention, while eliminating, for purposes of clarity, other elements. Those of ordinary skill in the art, upon considering the present description of the invention, will recognize that other elements and/or limitations can be desirable in order to implement some embodiments of the present invention. However, because such other elements and/or limitations can be readily ascertained by one of ordinary skill upon considering the present description, and are not necessary for a complete understanding of the present invention, a discussion of such elements and limitations is not provided herein.

It is worth mentioning that throughout the following description, when the article "a" is used to introduce an element, it does not have the meaning of "only one" it rather means of "one or more".

Certain terms used in this application and their meanings as used in this context are set forth at the outset for ease of reference. To the extent a term used herein is not defined below, it should be given the broadest definition persons in the pertinent art have given that term as reflected in at least one printed publication or issued patent. Further, the present invention is not limited by the usage of the terms shown below, as all equivalents, synonyms, new developments, and terms or techniques that serve the same or a similar purpose are considered to be within the scope of the present invention.

According to an aspect of the invention, there is provided a dental tray molding kit for making a custom impression tray. The kit includes a generic tray and a thermoformable material removably insertable in the generic tray and biteable by a user upon placement of the thermoformable material inserted into the generic tray in a mouth of the user.

As used herein, the expression "dental tray", also often referred to as a "dental impression tray" is intended to refer to a receptacle or device that can be used to carry impression material to the mouth, confine the material in apposition to the surfaces to be recorded, and control the impression material while it sets to form the impression.

As used herein, the expression "custom impression tray" is intended to refer to the result of applying a dental tray to teeth, thus producing a tray including an impression of the teeth on which the dental tray was applied.

As used herein, the expression "generic tray" is intended to refer to a generic dental tray for creating teeth impressions. As explained hereinbelow, the generic tray can either be a moldable generic tray that can form a first custom impression tray, or the generic tray can alternately be a rigid generic tray that requires the additional use of impression material in order to be able to produce a first custom impression tray

As used herein, the expression "thermoformable material" is intended to refer to refer to a material that can be manufactured using a thermoforming process, in which plastic can be heated to a pliable forming temperature, formed to a specific shape in a mold and trimmed to create a usable product.

As used herein, the expression "impression material" is intended to refer to a material that can be used in various areas of dentistry that is capable of receiving a dental impression and the area that the impression material covers.

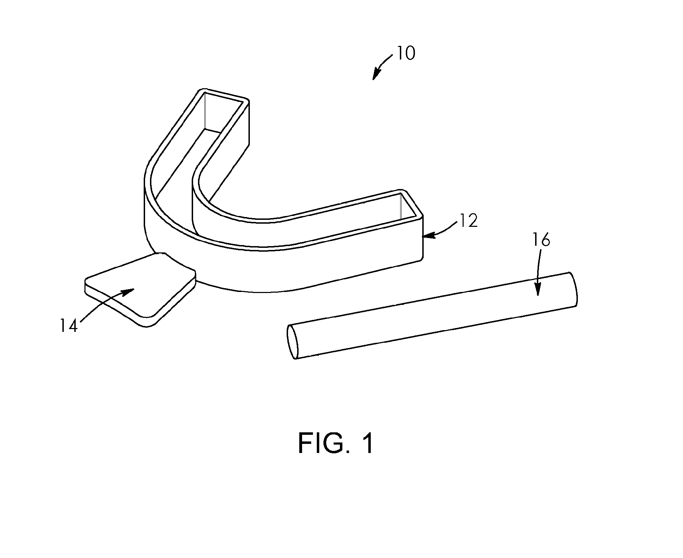

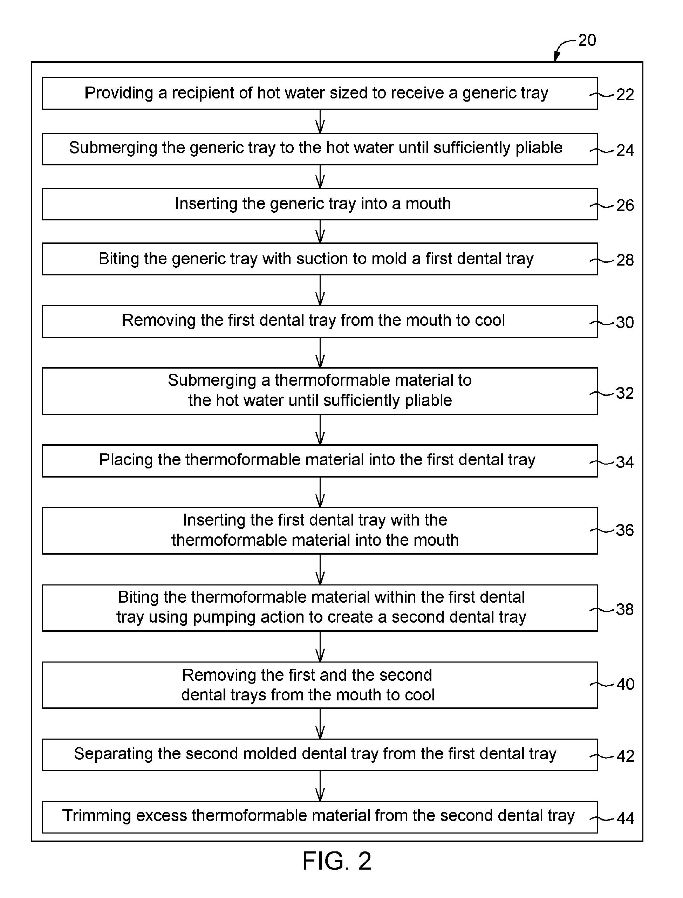

Referring to Figures 1 and 2, there is shown an embodiment of the dental tray molding kit 10, using a moldable generic tray 12. The moldable generic tray 12 is generally of a horseshoe shape with an open U-shaped cross-section. In some embodiments, the moldable generic tray 12 can have a handgrip 14 protruding from the moldable generic tray 12 to assist with handling. Preferably, the moldable generic tray 12 is made of an ethylene vinyl-acetate material. The moldable generic tray 12 is also typically sufficiently pliable for receiving a dental impression.

Depending on the user's dental development, the moldable generic tray 12 can also be sized to conform to the user's primary or permanent teeth.

The kit 10 also includes a thermoformable material 16 removably insertable in the moldable generic tray 12 and biteable by a user upon placement of the thermoformable material 16 inserted into the moldable generic tray 12 in a mouth of the user. In some embodiments, the thermoformable material 16 can be of a cylindrical shape.

Preferably, the thermoformable material 16 is a thermoplastic material. Also preferably, the thermoplastic material is a polyester material. The polyester material can be derived from a caprolactone monomer with a monomer content of about 0.5% Max, a water content of about 0.35% Max, a mean molecular weight of about 80 000, a melting point of about 58°C to 60°C, an elongation at break of between about 500% to 800%, a bending modulus of 280 MPa, a tensile strength of about 23 MPa and/or a melt index about 3 to 5 g/10 min (160°C). The thermoformable material is also typically sufficiently pliable for receiving a dental impression. In one preferred embodiment of the present invention, the thermoplastic material is polycaprolactone, similar to the material used as a thermosetting material in US 5,213,498.

The kit 10 can further include instructions. The instructions can be a dental tray molding method 20. The method 20 can include providing 22 a recipient of hot water sized to receive the moldable generic tray 12. Preferably, the hot water is boiling water or is at least at a temperature for softening the moldable generic tray 12. The method 20 also can include submerging 24 the moldable generic tray 12 to the boiling water until sufficiently pliable and inserting 26 the moldable generic tray 12 into the mouth of the user.

The method 20 can include biting 28 the moldable generic tray 12 with suction to mold a first custom impression tray. The molding of the moldable generic tray 12 to the user's teeth can be achieved by pressing the moldable generic tray 12 with fingers and using suction. The suction can be a sucking action produced by the user.

The method 20 can include removing 30 the first custom impression tray from the mouth to cool. In some embodiments, the method 20 can further include a step of cooling the first custom impression tray by submerging the first custom impression tray to cold or ice water to set the first custom impression tray.

The method 20 can include submerging 32 the thermoformable material 16 in hot water until sufficiently pliable.

Preferably, the method 20 includes a step of applying orthodontic wax, petroleum jelly or a suitable material on and between the user's teeth to block embrasures, undercuts and black triangle. As used herein, the expression "black triangle" is intended to refer to spaces or embrasures between teeth that are not blocked by gingival papillae. The step of applying orthodontic wax can prevent complications from the thermoformable material hardening in spaces between teeth and thus difficult to retrieve.

The method 20 can also include placing 34 the thermoformable material 16 into the first custom impression tray and inserting 36 the first custom impression tray with the thermoformable material 16 in the mouth.

The method 20 can include biting 38 the thermoformable material 16 within the first custom impression tray using pumping action to create a second custom impression tray. The pumping action can be produced by the user using an up and down pumping action with the user's teeth until the thermoformable material 16 hardens within the user's mouth.

The method 20 can include removing 40 the first and the second custom impression trays from the mouth to cool. In some embodiments, the method 20 can further include a step of cooling the second custom impression tray by submerging the second custom impression tray to cold or ice water to set the second custom impression tray.

The method 20 can include separating 42 the second custom impression tray from the first custom impression tray and optionally, trimming 44 excess thermoformable material 16 from the second custom impression tray. Generally, trimming 44 can be performed using a knife, scissors or a razor blade.

Preferably, the kit 10 is configurable to whiten teeth of the user as a cosmetic procedure.

Advantageously, the dental tray molding method according to embodiments of the invention allows for a well-adapted custom mouth tray that holds the thermoformable material in place while creating the second custom impression tray and allows for easy manipulation of the thermoformable material for creating a thin, durable second custom impression tray having a precise dental fit.

Also advantageously, the dental tray molding method according to embodiments of the invention can be easily fabricated in a few minutes without having to send dental impression to a laboratory. The custom impression tray created is also generally thin and aesthetic and is not as noticeable as bulky dental trays. It is also well adapted to hold onto a user's teeth without requiring support or additional handling.

According to another embodiment of the invention, there is provided a dental tray molding kit, including a rigid generic tray; an impression material removably insertable in the rigid generic tray and biteable by a user upon placement of the impression material inserted into the rigid generic tray in a mouth of the user to create a first custom impression tray; and a thermoformable material removably insertable in the first custom impression tray and biteable by the user upon placement of the thermoformable material inserted into the first custom impression tray in the mouth.

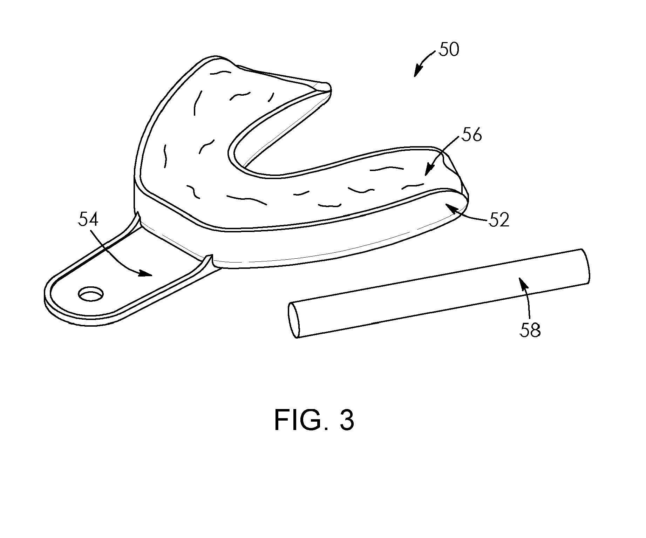

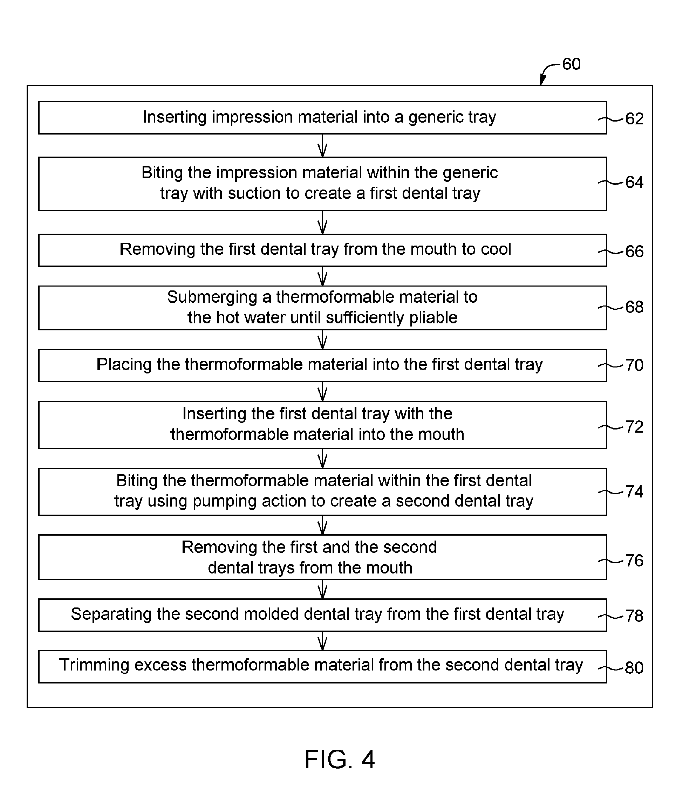

Referring now to Figures 3 and 4, there is shown an embodiment of the dental tray molding kit 50. The kit 50 includes a rigid generic tray 52. In some embodiments, the rigid generic tray 52 can have a handgrip 54 protruding from the generic tray 52 to assist with handling. Preferably, the rigid generic tray 52 is made of plastic or metal.

Depending on the user's dental development, the rigid generic tray 52 can also be sized to conform to the user's primary or permanent teeth.

The kit 50 also includes an impression material 56 removably insertable in the rigid generic tray 52 and biteable by a user upon placement of the impression material 56 inserted into the rigid generic tray 52 in a mouth of the user to create a first custom impression tray. Preferably, the impression material 56 is obtained by mixing a putty base with a catalyst for about 30 to 45 seconds. The impression material 56 can be made of a polyether, a polysulfide, a hydrocolloid, a polyvinyl, a vinyl-polyether hydride, a condensation cured silicone or an addition silicone. The addition silicone can be a siloxane, a polysiloxane or a polyvinyl siloxane.

The kit 50 also includes a thermoformable material 58 removably insertable in the first custom impression tray and biteable by the user upon placement of the thermoformable material 58 inserted into the first custom impression tray in the mouth. Preferably, the thermoformable material 58 is a thermoplastic material. Also preferably, the thermoplastic material is a polyester material. The polyester material can be derived from a caprolactone monomer with a monomer content of about 0.5% Max, a water content of about 0.35% Max, a mean molecular weight of about 80 000, a melting point of about 58°C to 60°C, an elongation at break of between about 500% to 800%, a bending modulus of 280 MPa, a tensile strength of about 23 MPa and/or a melt index about 3 to 5 g/10 min (160°C). The thermoformable material is also typically sufficiently pliable for receiving a dental impression.

The kit 50 can further include instructions. The instructions can be a dental tray molding method 60. The method 60 can include inserting 62 the impression material 56 into the rigid generic tray 52.

The method 60 can include biting 64 the impression material 56 within the rigid generic tray 52 for about 60 seconds with suction until the impression material 56 sets to mold a first custom impression tray. The molding of the rigid generic tray 52 to the user's teeth can be achieved by pressing the rigid generic tray 52 with fingers and using suction. The suction can be a sucking action produced by the user.

The method 60 can include removing 66 the first custom impression tray from the mouth. The method 60 can also include submerging 68 the thermoformable material 58 to the hot water until sufficiently pliable.

Preferably, the method 20 includes a step of applying orthodontic wax, petroleum jelly or a suitable material on and between the user's teeth to block embrasures, undercuts and black triangle.

The method 60 can also include placing 70 the thermoformable material 58 into the first custom impression tray and inserting 72 the first custom impression tray with the thermoformable material 58 in the mouth.

The method 60 can include biting 74 the thermoformable material 58 within the first custom tray using pumping action to create a second custom impression tray. The pumping action can be produced by the user using an up and down pumping action with the user's teeth until the thermoformable material 58 hardens within the user's mouth.

The method 60 can include removing 76 the first and the second custom impression trays from the mouth to cool. In some embodiments, the method 60 can further include a step of cooling the second custom impression tray by submerging the second custom impression tray to cold or ice water to set the second custom impression tray.

The method 60 can include separating 78 the second custom impression tray from the first custom impression tray and optionally, trimming 80 excess thermoformable material 58 from the second custom impression tray. Generally, trimming 80 can be performed using a knife, scissors or a razor blade.

In some embodiments of the present invention, the method 60 can include, between the step of applying a blocking material and the step of inserting the first custom impression tray with the thermoformable material in the mouth, the step of placing a thin separating sheet over the thermoformable material. For example, after the blocking wax is applied to teeth and after the polycaprolactone thermoplastic cylinder is placed in either the first custom impression tray or the impression held by the rigid tray, a thin separating sheet can be placed over the thermoplastic polycaprolactone cylinder and then bit into to form the second custom impression tray. This can ensure that the second custom impression tray is still a tight fit but can be even more easily removed.

Preferably, the kit 50 is configurable to whiten teeth of the user as a cosmetic procedure.

Advantageously, the dental tray molding method according to embodiments of the invention allows for a well-adapted custom mouth tray that holds the thermoformable material in place while creating the second custom impression tray and allows for easy manipulation of the thermoformable material for creating a thin, durable second custom impression dental tray having a precise dental fit.

Also advantageously, the dental tray molding method according to embodiments of the invention can be easily fabricated in a few minutes without having to send a dental impression to a laboratory. The custom impression tray created is also generally thin and aesthetic and is not as noticeable as bulky dental trays. It is also well adapted to hold onto a user's teeth without requiring support or additional handling.

Some embodiments of the methods are especially well adapted for:
- a teeth whitening procedure in which the second custom impression dental tray allows for close contact of hydrogen and carbamide peroxide with teeth;
- an anti-cavity procedure to strengthen teeth enamel and dentine in which the second custom impression dental tray allows for fluoride, potassium nitrate and calcium in a basic PH+ gel to be applied to teeth to remineralize enamel and dentine, reverse decalcification and early stages of decay to strengthen dental structure;
- a de-sensitizing procedure in which the second custom impression dental tray contains fluoride and potassium nitrate;
- an anti-bruxism procedure in which the second custom impression dental tray is worn to protect against teeth grinding; or
- an orthodontic procedure in which the second custom impression dental tray maintains proper alignment of teeth and prevents further teeth shifting when worn during sleep.

Advantageously, in procedures requiring the use of a gel, the second custom impression tray can be in close contact with a user's teeth without the gel leaking and can be hard enough to be used as a bruxism device, in accordance with some embodiments of the methods.

While this invention has been particularly shown and described with references to preferred embodiments thereof, it will be understood by those skilled in the art that various changes in form and details can be made therein without departing from the scope of the invention encompassed by the appended claims.

## Claims

1. A dental tray molding kit for making a custom impression tray, the kit comprising:
- a generic tray; and
- a thermoformable material removably insertable in the generic tray and biteable by a user upon placement of the thermoformable material inserted into the generic tray in a mouth of the user,
wherein the generic tray is selected from the group comprising:
a moldable generic tray; and
a combination of a rigid generic tray and an impression material removably insertable in the rigid generic tray and biteable by a user upon placement of the impression material inserted into the rigid generic tray in a mouth of the user to create a custom impression tray, and wherein the thermoformable material is removably insertable in the custom impression tray and biteable by the user upon placement of the thermoformable material inserted into the impression tray in the mouth.

2. The dental tray molding kit according to claim 1, wherein the generic tray is the moldable generic tray, preferably made of an ethylene vinyl-acetate material.

3. The dental tray molding kit according to claim 2, wherein the moldable generic tray is sufficiently pliable for receiving a dental impression.

4. The dental tray molding kit according to any one of claims 1 to 3, wherein the thermoformable material is a thermoplastic material, preferably a polyester material.

5. The dental tray molding kit according to any one of claims 1 to 4, wherein the thermoplastic material is polycaprolactone.

6. The dental tray molding kit according to any one of claims 1 to 5, wherein the generic tray is the combination of the rigid generic tray; and the impression material removably insertable in the rigid generic tray and biteable by the user upon placement of the impression material inserted into the rigid generic tray in the mouth of the user to create the custom impression tray.

7. The dental tray molding kit according to claim 6, wherein the impression material is selected from the group comprising a polyether, a polysulfide, a hydrocolloid, a polyvinyl, a vinyl-polyether hydride, a condensation cured silicone, an addition silicone, a siloxane, a polysiloxane and a polyvinyl siloxane.

8. The dental tray molding kit according to any one of claims 1 to 7, wherein the thermoformable material has a cylindrical shape.

9. The dental tray molding kit according to any one of claims 1 to 8, further comprising instructions for use of the kit, preferably to whiten teeth of the user as a cosmetic procedure.

10. A dental tray molding method comprising the steps of:
- providing a recipient of hot water sized to receive a moldable generic tray;
- submerging the moldable generic tray in hot water until sufficiently pliable;
- inserting the moldable generic tray into a mouth;
- biting the moldable generic tray with suction to mold a first custom impression tray;
- removing the first custom impression tray from the mouth to cool;
- submerging a thermoformable material in the hot water until sufficiently pliable;
- placing the thermoformable material into the first custom impression tray;
- inserting the first custom impression tray with the thermoformable material in the mouth;
- biting the thermoformable material within the first custom impression tray using pumping action to create a second custom impression tray;
- removing the first and the second custom impression trays from the mouth to cool; and
- separating the second custom impression dental tray from the first custom impression tray.

11. The method according to claim 10, further comprising, before the step of inserting the first custom impression tray with the thermoformable material in the mouth, the step of:
- applying a blocking material between teeth in the mouth to block at least one of embrasures, undercuts and black triangles.

12. The method according to claim 11, wherein the blocking material is selected from the group comprising orthodontic wax and petroleum jelly.

13. The method according to any one of claims 10 to 12, further comprising, after the step of removing the first and the second custom impression trays from the mouth to cool, the step of:
- cooling the second custom impression tray by submerging the second custom impression tray in a cooling fluid to set the second impression tray.

14. The method according to any one of claims 10 to 13, further comprising, after the step of separating the second custom impression tray from the first custom impression tray, the step of:
- trimming excess thermoformable material from the second custom impression tray.

15. The method according to any one of claims 10 to 14, further comprising, between the step of applying a blocking material and the step of inserting the first custom impression tray with the thermoformable material in the mouth, the step of:
- placing a thin separating sheet over the thermoformable material.
